# EUROPEAN PATENT APPLICATION

(11) **EP 1 780 271 A1**
(43) Date of publication of application: **02.05.2007**
(21) Application number: 06022442.5
(22) Date of filing: 26.10.2006
(51) Int. Cl.: C12N 11/04, C02F 3/00

(54) **Entrapping immobilization pellets of anaerobic ammonium oxidizing bacteria and process for producing the same**

(30) Priority: 01.11.2005 JP 2005318412
(71) Applicant: Hitachi Plant Technologies, Ltd., Tokyo (JP)
(72) Inventor: Isaka, Kazuichi, Chiyoda-ku, Tokyo (JP); Sumino, Tatsuo, Chiyoda-ku, Tokyo (JP); Noto, Kazuhiko, Chiyoda-ku, Tokyo (JP)
(74) Representative: Grünecker, Kinkeldey, Stockmair & Schwanhäusser Anwaltssozietät

(57) **Abstract**

There is provided entrapping immobilization pellets having high activity with a smaller amount of cells of anaerobic ammonium oxidizing bacteria. The entrapping immobilization pellets (14), in which anaerobic ammonium oxidizing bacteria are entrapped and immobilized in an immobilizing material, comprise fine activated carbon powder and/or fine metal powder such as magnetite or iron powder.

## Description

### BACKGROUND OF THE INVENTION

### Field of the Invention

The present invention relates to entrapping immobilization pellets and a process for producing the same. More particularly, the present invention relates to entrapping immobilization pellets in which anaerobic ammonium oxidizing bacteria are entrapped and immobilized and a process for producing the same, and wastewater treatment equipment comprising the entrapping immobilization pellets.

### Description of the Related Art

Ammonium nitrogen contained in wastewater is a substance that causes eutrophication in rivers, lakes, and seas, and must be efficiently removed in a wastewater treatment process.

Various denitrification processes for biologically removing ammonium nitrogen have generally been known. The most common example of such denitrification processes is a process for removing ammonium nitrogen comprising aerobically nitrifying ammonium into nitrite or nitrate by nitrifying bacteria, and then anaerobically denitrifying the nitrite or nitrate by denitrifying bacteria.

In such denitrification treatment, an organic substance must be added to nitrite or nitrate for denitrification reaction at a concentration of about three times the total nitrogen concentration, because denitrifying bacteria are heterotrophic. In addition, this denitrification process needs a large amount of oxygen for nitrification reaction and thus involves a high running cost, disadvantageously.

Anaerobic ammonium oxidation has been proposed in recent years as a device to solve these problems, in order to carry out biological denitrification treatment efficiently and inexpensively. This process comprises first converting a part of ammonium into nitrite in nitrification reaction represented by the formula (1), for example, and then denitrifying the remaining ammonium and the produced nitrite by anaerobic ammonium oxidizing bacteria in denitrification reaction represented by the formula (2).

NH₄⁺ + 1.5O₂ + 2NaOH → NaNO₂ + Na⁺ + 3H₂O (1)

NH₄⁺ + 1.31NO₂ + 0.0425CO₂ → 1.045N₂ + 0.22NO₃⁻ + 1.87H₂O + 0-09OH⁻ + 0.0425CH₂O (2)

This process converts only a part of ammonium into nitrite, and thus can considerably reduce the amount of oxygen necessary for nitrification reaction. Further, this process does not need an organic substance for denitrification reaction because anaerobic ammonium oxidizing bacteria are autotrophic, and therefore it is said that the process can denitrify ammonium-containing water inexpensively.

For example, Japanese Patent Application Laid-Open No. 2003-53385 discloses an entrapping immobilization process comprising entrapping and immobilizing anaerobic ammonium oxidizing bacteria in a polymer gel as such a denitrification process. This process entraps and immobilizes anaerobic ammonium oxidizing bacteria in a polymer gel and packs a treatment tank with the entrapping immobilization pellets, and therefore it is said that the process can retain anaerobic ammonium oxidizing bacteria in a treatment tank stably and densely.

Japanese Patent Application Laid-Open No. 2003-235554 discloses a process for producing entrapping immobilization pellets comprising mixing nitrifying bacteria with activated carbon and then mixing a gelling material with the mixture to form a gel. It is said that the process can prevent inactivation or killing of nitrifying bacteria in the gelling step.

However, only an extremely small amount of anaerobic ammonium oxidizing bacteria are naturally occurring, and anaerobic ammonium oxidizing bacteria grow only at an extremely low specific growth rate. The problem is, in particular, that it is difficult to obtain cells enough to prepare a large amount of entrapping immobilization pellets.

Specifically, it is necessary to reduce the amount of cells of anaerobic ammonium oxidizing bacteria to be entrapped and immobilized as much as possible and maintain high activity of entrapping immobilization pellets, since it is difficult to obtain cells of anaerobic ammonium oxidizing bacteria to be entrapped and immobilized.

The process of Japanese Patent Application Laid-Open No. 2003-53385 has a drawback in that entrapping immobilization pellets must contain a large amount of cells of anaerobic ammonium oxidizing bacteria at a VSS concentration of about 6,000 mg/L (this estimated value is based on the description in the specification that a tank has a packing ratio of 50% and a VSS concentration of 3,000 mg/L), because entrapping immobilization pellets do not exhibit activity even after acclimatization when the amount of cells of anaerobic ammonium oxidizing bacteria is reduced.

The process has another drawback in that anaerobic ammonium oxidizing bacteria are sedimented in the step of gelling entrapping immobilization pellets, making it difficult to uniformly disperse the bacteria in a gel, because anaerobic ammonium oxidizing bacteria tend to be sedimented extremely easily.

Japanese Patent Application Laid-Open No. 2003-235554 does not examine anaerobic ammonium oxidizing bacteria.

The present invention has been achieved in view of such circumstances. An object of the present invention is to provide entrapping immobilization pellets having high activity with a smaller amount of cells of anaerobic ammonium oxidizing bacteria, a process for producing the same, and wastewater treatment equipment comprising the entrapping immobilization pellets.

### SUMMARY OF THE INVENTION

To attain the aforementioned object, a first aspect of the present invention provides entrapping immobilization pellets in which anaerobic ammonium oxidizing bacteria are entrapped and immobilized in an immobilizing material, the entrapping immobilization pellets comprising fine activated carbon powder and/or fine metal powder such as magnetite or iron powder.

According to the present invention, entrapping immobilization pellets in which anaerobic ammonium oxidizing bacteria are entrapped and immobilized comprise fine activated carbon powder and/or fine metal powder such as magnetite or iron powder. Thus, entrapping immobilization pellets having high activity can be produced with a smaller amount of cells of anaerobic ammonium oxidizing bacteria.

The activated carbon may be particulate activated carbon or granular activated carbon, and is particularly preferably powdered activated carbon.

A second aspect of the present invention provides the entrapping immobilization pellets according to the first aspect, comprising cells of the anaerobic ammonium oxidizing bacteria at a VSS concentration of 100 mg/L to 3,000 mg/L.

According to the second aspect, the entrapping immobilization pellets comprise cells of the anaerobic ammonium oxidizing bacteria at a VSS concentration of 100 mg/L to 3,000 mg/L. Thus, entrapping immobilization pellets having high activity can be produced with a smaller amount of cells of anaerobic ammonium oxidizing bacteria.

The entrapping immobilization pellets preferably comprise cells of the anaerobic ammonium oxidizing bacteria at a VSS concentration of 100 mg/L to 1,000 mg/L.

The VSS concentration is determined by a known determination method (see "Sewage Testing Method", published by Japan Sewage Works Association, 1997 edition, p.271, for example). Specifically, the VSS concentration is an amount of an organic substance in sludge determined on a weight basis and represents an amount of microbial cells.

A third aspect of the present invention provides the entrapping immobilization pellets according to the first or second aspect, comprising the fine activated carbon powder and/or the fine metal powder such as magnetite or iron powder in an amount of 0.05 mass% to 5 mass% based on the entrapping immobilization pellets.

The present inventors have defined that the fine activated carbon powder and/or the fine metal powder such as magnetite or iron powder are added to the entrapping immobilization pellets in an amount of 0.05 mass% to 5 mass%. Thus, entrapping immobilization pellets having high activity can be produced with a smaller amount of cells of anaerobic ammonium oxidizing bacteria.

The amount of the fine powder added is preferably 0.1 mass% to 5 mass%, and more preferably 0.5 mass% to 5 mass%.

To attain the aforementioned object, a fourth aspect of the present invention provides a process for producing entrapping immobilization pellets in which anaerobic ammonium oxidizing bacteria are entrapped and immobilized in an immobilizing material, the process comprising mixing the anaerobic ammonium oxidizing bacteria with fine activated carbon powder and/or fine metal powder such as magnetite or iron powder, and then mixing the immobilizing material with the mixture to form a gel.

According to the present invention, the anaerobic ammonium oxidizing bacteria are entrapped and immobilized by mixing the anaerobic ammonium oxidizing bacteria with the fine activated carbon powder and/or the fine metal powder such as magnetite or iron powder, and then mixing the immobilizing material with the mixture to form a gel. Thus, entrapping immobilization pellets having high activity can be produced with a smaller amount of cells of anaerobic ammonium oxidizing bacteria.

The activated carbon may be particulate activated carbon or granular activated carbon, and is particularly preferably powdered activated carbon.

A fifth aspect of the present invention provides the process for producing entrapping immobilization pellets according to the fourth aspect, wherein the entrapping immobilization pellets comprise cells of the anaerobic ammonium oxidizing bacteria at a VSS concentration of 100 mg/L to 3,000 mg/L, and comprise the fine activated carbon powder and/or the fine metal powder such as magnetite or iron powder in an amount of 0.05 mass% to 5 mass% based on the entrapping immobilization pellets.

According to the fifth aspect, the entrapping immobilization pellets comprise cells of the anaerobic ammonium oxidizing bacteria at a VSS concentration of 100 mg/L to 3,000 mg/L, and comprise the fine activated carbon powder and/or the fine metal powder such as magnetite or iron powder in an amount of 0.05 mass% to 5 mass% based on the entrapping immobilization pellets. Thus, entrapping immobilization pellets having high activity can be produced with a smaller amount of cells of anaerobic ammonium oxidizing bacteria.

A sixth aspect of the present invention provides wastewater treatment equipment comprising a biological treatment tank packed with the entrapping immobilization pellets according to any one of the first to third aspects.

According to the sixth aspect, the wastewater treatment equipment comprises a biological treatment tank packed with the entrapping immobilization pellets of the present invention. Thus, wastewater can be efficiently treated with a smaller amount of cells of anaerobic ammonium oxidizing bacteria.

According to the present invention, entrapping immobilization pellets having high activity can be obtained with a smaller amount of cells of anaerobic ammonium oxidizing bacteria.

### BRIEF DESCRIPTION OF THE DRAWINGS

Fig. 1 is a view describing the operation flow of the process for producing entrapping immobilization pellets which is an embodiment of the present invention;
Fig. 2 is a view describing the wastewater treatment equipment of the examples; and
Fig. 3 is a graph showing the results of the wastewater treatment test in the examples.

### DETAILED DESCRIPTION OF THE PREFERRED EMBODIMENTS

Preferred embodiments of the entrapping immobilization pellets and the process for producing the same, and the wastewater treatment equipment comprising the entrapping immobilization pellets according to the present invention will be described below in detail with reference to the accompanying drawings.

The present inventors have found that, in the process of forming entrapping immobilization pellets, anaerobic ammonium oxidizing bacteria can effectively grow and activity of entrapping immobilization pellets comprising the anaerobic ammonium oxidizing bacteria can be effectively increased, in particular, by allowing the entrapping immobilization pellets to contain fine activated carbon powder and/or fine metal powder such as magnetite or iron powder. The present invention has been accomplished based on this finding.

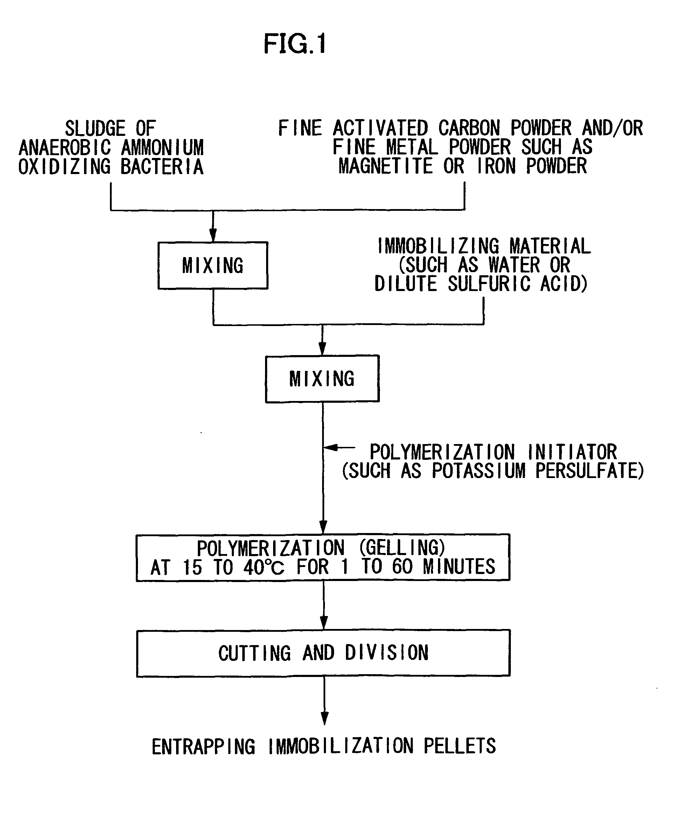

Fig. 1 is a view describing the operation flow of the process for producing entrapping immobilization pellets which is an embodiment of the present invention.

As shown in Fig. 1, sludge of anaerobic ammonium oxidizing bacteria to be entrapped and immobilized is first mixed with fine activated carbon powder and/or fine metal powder such as magnetite or iron powder, and then the mixture is mixed with an immobilizing material and sufficiently stirred to prepare a raw material solution. In this case, water or dilute sulfuric acid as a reaction control agent is added to the immobilizing material.

Next, a polymerization initiator such as potassium persulfate is added to the raw material solution to cause polymerization reaction, so that the immobilization material is gelled and the sludge of anaerobic ammonium oxidizing bacteria is entrapped and immobilized therein. Here, polymerization (gelation) is carried out at a polymerization temperature of 15°C to 40°C, and preferably 20°C to 30°C, for a polymerization time of 1 to 60 minutes, and preferably 1.5 to 60 minutes.

Then, the gelled pellet is divided into about 3 mm-square angular pellets. The entrapping immobilization pellets of the present invention are thus produced.

Conventionally, entrapping immobilization pellets have been produced by mixing microorganisms with an immobilizing material, and then gelling the mixture in a gelling step such as polymerization reaction or freezing or defrosting treatment. However, in the present invention, it is important to first mix anaerobic ammonium oxidizing bacteria with fine activated carbon powder and/or fine metal powder such as magnetite or iron powder (hereinafter sometimes collectively referred to "fine powder"), and then mix an immobilizing material with the mixture to cause polymerization reaction. This is because the fine powder tends to have decreased ability to adsorb the anaerobic ammonium oxidizing bacteria or a substance that is released from the anaerobic ammonium oxidizing bacteria, for example, when the immobilizing material is first mixed with the fine powder.

Examples of anaerobic ammonium oxidizing bacteria used to be entrapped and immobilized include those concentrated and separated by culturing or the like; and substances containing various microorganisms such as activated sludge in sewage treatment plants, sludge in lakes, rivers, or sea, or soil.

Anaerobic ammonium oxidizing bacteria are preferably cultured by a process of causing the bacteria to adhere to a nonwoven cloth (see So IKUTA, Kazuichi ISAKA, and Tatsuo SUMINO (2004), "Acclimatization of Anaerobic Ammonium Oxidizing Bacteria in Continuous Culture Systems", Lectures and Papers, 38th Annual Meeting of Japan Society on Water Environment, p.372) or a filter bed to culture the bacteria, and stripping the biofilm therefrom for use. The entrapping immobilization pellets of the present embodiment comprise cells of anaerobic ammonium oxidizing bacteria at a VSS concentration of preferably 100 mg/L to 3,000 mg/L, and more preferably 100 mg/L to 1,000 mg/L.

The entrapping immobilization pellets of the present invention comprise fine activated carbon powder and/or fine metal powder such as magnetite or iron powder. Particularly preferably, the pellets comprise activated carbon. The entrapping immobilization pellets may comprise one of these powders or a combination of two or more thereof.

The activated carbon may be particulate activated carbon or granular activated carbon, and is particularly preferably powdered activated carbon.

The fine powder preferably has an average particle size of 50 µm or less. If the fine powder has an average particle size of more than 50 µm, the fine powder may be sedimented in the gelling step, making it difficult to uniformly disperse anaerobic ammonium oxidizing bacteria.

The amount of the fine powder added is preferably 0.05 mass% to 5 mass%, more preferably 0.1 mass% to 5 mass%, and particularly preferably 0.5 mass% to 5 mass% based on the entrapping immobilization pellets. If the amount of the fine powder is less than 0.05 mass%, the effect of improving denitrification activity of the entrapping immobilization pellets tends to be small. If the amount of the fine powder is more than 5 mass%, the entrapping immobilization pellets tend to have reduced pellet strength. It is therefore preferable to appropriately select the amount of the fine powder depending on the type of the fine powder used.

Examples of the immobilizing material include monomers, prepolymers, and oligomers. These materials are roughly classified into naturally occurring immobilizing materials such as agar and alginic acid, and synthetic immobilizing materials such as polyethylene glycol, polyvinyl alcohol, and polyacrylamide. It is difficult to use a naturally occurring immobilizing material for wastewater treatment, because the resulting gel of entrapping immobilization pellets itself is highly biodegradable and thus cannot be used for a long time, and has low pellet strength. Accordingly, it is preferable to use a synthetic immobilizing material that has sufficient pellet strength and is poorly biodegradable.

As such a synthetic immobilizing material, a polyethylene glycol polymer or polyvinyl alcohol polymer may be preferably used, for example. Specifically, a prepolymer having a molecular weight of 4,000 which contains ethylene oxide and propylene oxide at 7:3 and has a diacrylate as a terminal group may be preferably used as such an immobilizing material, for example.

Further, polyethylene glycol acrylate, polyethylene glycol diacrylate, polyethylene glycol methacrylate, or the like may be used as such an immobilizing material. In addition, the following prepolymers may also be used:
monomethacrylates such as polyethylene glycol monomethacrylate, polyprene glycol monomethacrylate, polypropylene glycol monomethacrylate, methoxydiethylene glycol methacrylate, methoxypolyethylene glycol methacrylate, methacryloyloxyethyl hydrogen phthalate, methacryloyloxyethyl hydrogen succinate, 3-chloro-2-hydroxypropyl methacrylate, stearyl methacrylate, 2-hydroxy methacrylate, and ethyl methacrylate;
monoacrylates such as 2-hydroxyethyl acrylate, 2-hydroxypropyl acrylate, isobutyl acrylate, t-butyl acrylate, isooctyl acrylate, lauryl acrylate, stearyl acrylate, isobornyl acrylate, cyclohexyl acrylate, methoxytriethylene glycol acrylate, 2-ethoxyethyl acrylate, tetrahydrofurfuryl acrylate, phenoxyethyl acrylate, nonylphenoxypolyethylene glycol acrylate, nonylphenoxypolypropylene glycol acrylate, silicon-modified acrylate, polypropylene glycol monoacrylate, phenoxyethyl acrylate, phenoxydiethylene glycol acrylate, phenoxypolyethylene glycol acrylate, methoxypolyethylene glycol acrylate, acryloyloxyethyl hydrogen succinate, and lauryl acrylate;
dimethacrylates such as 1,3-butylene glycol dimethacrylate, 1,4-butanediol dimethacrylate, ethylene glycol dimethacrylate, diethylene glycol dimethacrylate, triethylene glycol dimethacrylate, polyethylene glycol dimethacrylate, butylene glycol dimethacrylate, hexanediol dimethacrylate, neopentyl glycol dimethacrylate, polyprene glycol dimethacrylate, 2-hydroxy-1,3-dimethacryloxypropane,
2,2-bis-4-methacryloxyethoxyphenylpropane,
3,2-bis-4-methacryloxydiethoxyphenylpropane, and
2,2-bis-4-methacryloxypolyethoxyphenylpropane;
diacrylates such as ethoxylated neopentyl glycol diacrylate, polyethylene glycol diacrylate, 1,6-hexanediol diacrylate, neopentyl glycol diacrylate, tripropylene glycol diacrylate, polypropylene glycol diacrylate, 2,2-bis-4-acryloxyethoxyphenylpropane, 2-hydroxy-1-acryloxy-3-methacryloxypropane;
trimethacrylates such as trimethylolpropane trimethacrylate;
triacrylates such as trimethylolpropane triacrylate, pentaerythritol triacrylate, trimethylolpropane EO-added triacrylate, glycerol PO-added triacrylate, and ethoxylated trimethylolpropane triacrylate;
tetraacrylates such as pentaerythritol tetraacrylate, ethoxylated pentaerythritol tetraacrylate, propoxylated pentaerythritol tetraacrylate, and ditrimethylolpropane tetraacrylate;
urethane acrylates such as urethane acrylate, urethane dimethyl acrylate, and urethane trimethyl acrylate; and
other compounds such as acrylamide, acrylic acid, and dimethylacrylamide.

The immobilizing materials may be used singly or in a combination of two or more.

Polymerization of the entrapping immobilization pellets is most appropriately radical polymerization using potassium persulfate, but may be polymerization using ultraviolet rays or electron beams or redox polymerization. In radical polymerization using potassium persulfate, potassium persulfate is preferably added in an amount of 0.001 to 0.25 mass%, and an amine polymerization accelerator is preferably added in an amount of 0.01 to 0.5 mass%. The amine polymerization accelerator is preferably, but is not limited to, β-dimethylaminopropionitrile or N,N,N',N'-tetramethylethylenediamine.

Sheet forming, tube forming, drop granulation, block forming, or the like may be used as a process for forming such entrapping immobilization pellets.

Preferably, the entrapping immobilization pellets produced above are activated (the anaerobic ammonium oxidizing bacteria are acclimatized) before full-scale operation of wastewater treatment. The entrapping immobilization pellets are preferably activated by a process of bringing real wastewater or synthetic wastewater (later-described synthetic wastewater in Table 1, for example) containing ammonium and nitrite into contact with the entrapping immobilization pellets.

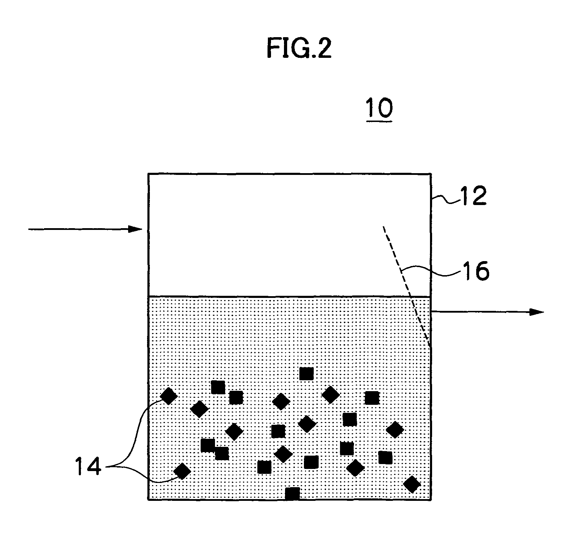

Next, an example of applying the entrapping immobilization pellets to wastewater treatment for removing nitrogen will be described. Fig. 2 is a view describing the wastewater treatment equipment 10 of the present embodiment.

The wastewater treatment equipment 10 in Fig. 2 comprises a biological treatment tank 12 packed with the entrapping immobilization pellets 14 of the present invention, and a filter 16 that prevents the entrapping immobilization pellets 14 from flowing out of the biological treatment tank 12. Thus, wastewater is fed to the biological treatment tank 12, denitrified by anaerobic ammonium oxidizing bacteria in the entrapping immobilization pellets 14 of the present invention, and discharged from the biological treatment tank 12 through the filter 16 as indicated by the arrows.

When the biological treatment tank 12 is packed with the entrapping immobilization pellets 14 of the present invention in this manner, wastewater can be effectively treated with even a small amount of cells of anaerobic ammonium oxidizing bacteria.

Next, there will be described a mechanism in which the entrapping immobilization pellets exhibit relatively high activity with even a small amount of cells of anaerobic ammonium oxidizing bacteria when fine activated carbon powder and/or fine metal powder such as magnetite or iron powder are added to the pellets. This mechanism is not limited to the following description, because the mechanism is not entirely clear.

It is generally known that anaerobic ammonium oxidizing bacteria do not start growing unless the bacteria have a certain cell concentration or higher. Accordingly, it is assumed that the bacteria mutually detect the cell concentration in some way, and start growing drastically when the bacteria have a certain cell concentration or higher.

Specifically, it is assumed that anaerobic ammonium oxidizing bacteria generate some substance, and detect the cell concentration by themselves based on the concentration of the substance. Presumably, when fine activated carbon powder and/or fine metal powder such as magnetite or iron powder are present in the entrapping immobilization pellets, the concentration of some substance generated from anaerobic ammonium oxidizing bacteria is locally increased due to the adsorption effect by the fine powder to make the anaerobic ammonium oxidizing bacteria detect the cell concentration incorrectly, so that the bacteria start growing even if the actual cell concentration is low.

The present inventors have discussed a mechanism of improving activity of the entrapping immobilization pellets by the presence of fine activated carbon powder and/or fine metal powder such as magnetite or iron powder as follows.

Specifically, polymerization of the immobilizing material tends to be locally inhibited, and the entrapping immobilization pellets tend to have relatively large voids near the fine activated carbon powder and/or the fine metal powder such as magnetite or iron powder. Thus, the surface area of the entrapping immobilization pellets is increased, and the substrate is easily diffused. Diffusion of the substrate is assumed to be an important factor to affect the activity of anaerobic ammonium oxidizing bacteria.

Anaerobic ammonium oxidizing bacteria have a characteristic of being extremely easily sedimented, and are easily sedimented and aggregated in the gelling step. However, the presence of fine activated carbon powder and/or fine metal powder such as magnetite or iron powder inhibits sedimentation or aggregation of anaerobic ammonium oxidizing bacteria and improves dispersibility of the bacteria.

According to the entrapping immobilization pellets and the process for producing the same and the wastewater treatment equipment comprising the entrapping immobilization pellets according to the present invention as described above, entrapping immobilization pellets having high activity can be obtained with a smaller amount of cells of anaerobic ammonium oxidizing bacteria.

The entrapping immobilization pellets and the process for producing the same according to the present invention are as described above. However, the present invention is not limited to the above embodiments, and various embodiments are possible.

For example, in the present embodiment described above, denitrification treatment is carried out by adding fine activated carbon powder and/or fine metal powder such as magnetite or iron powder to entrapping immobilization pellets of anaerobic ammonium oxidizing bacteria. The present invention can also be applied to a case where denitrification treatment is carried out by anaerobic ammonium oxidizing bacteria cultured (grown) or a case where denitrification treatment is carried out by anaerobic ammonium oxidizing bacteria not entrapped and immobilized.

### Examples

Next, the present invention will be described in more detail below with reference to examples. However, the present invention is not limited to the following examples.

In the present examples, the following materials were used for entrapping immobilization pellets. Entrapping immobilization pellets were produced according to the above-described operation flow in Fig. 1.

### (Materials for entrapping immobilization pellets)

Activated sludge: Sludge of anaerobic ammonium oxidizing bacteria
Fine powder: Coconut shell-based powdered activated carbon (average particle size: 20 µm)
Immobilizing material: Polyethylene glycol methacrylate, 10 mass%
Polymerization initiator: Potassium persulfate, 0.025 mass%
Polymerization accelerator: N,N,N',N'-tetramethylethylenediamine, 0.050 mass%

First, 1,000 mg/L of the sludge of anaerobic ammonium oxidizing bacteria (hereinafter referred to as anaerobic ammonium oxidizing bacteria) was mixed with 1 g of the powdered activated carbon having an average particle size of 20 µm per 100 g of entrapping immobilization pellets (corresponding to about 1 mass%). Then, polyethylene glycol methacrylate was mixed with the mixture to prepare a raw material solution.

Next, potassium persulfate and the crosslinking agent in Table 1 were added to the raw material solution, and the mixture was polymerized at a polymerization temperature of 20°C for 0.5 hour. The polymer was divided into 3 mm-square approximately cubic forms to prepare entrapping immobilization pellets of Example 1 (gel concentration: 10 mass%).

Entrapping immobilization pellets of Example 2 were prepared in the same manner as in Example 1, except that magnetite (2 g per 100 g of the entrapping immobilization pellets, average particle size: 20 µm) was used instead of the powdered activated carbon.

Entrapping immobilization pellets of Comparative Example 1 were prepared in the same manner as in Example 1, except that the powdered activated carbon was not added.

Each biological treatment tank 12 shown in Fig. 2 was packed with the entrapping immobilization pellets of Examples 1 or 2 or the entrapping immobilization pellets of Comparative Example 1 prepared as above, respectively, at a packing ratio of 20%, and the tank was operated for wastewater treatment (the anaerobic ammonium oxidizing bacteria were acclimatized) under the following treatment conditions over four months. Then, denitrification activity of the respective entrapping immobilization pellets was determined.

Denitrification activity was evaluated by determination of the total nitrogen concentration (mg/L) in wastewater by ion chromatography. The evaluation results at that time are shown in Table 2.

### (Treatment conditions)

### Wastewater used: Synthetic wastewater

**[Table 1]**

| Substance | Concentration | Unit |
|---|---|---|
| NaNO₂ | 50~250 asN | mg/L |
| (NH₄)SO₄ | 50~210 asN | mg/L |
| KHCO₃ | 500 | mg/L |
| KH₂PO₄ | 27 | mg/L |
| MgSO₄.7H₂O | 300 | mg/L |
| CaCl₂.2H₂O | 180 | mg/L |
| T. Element S1 | 1 | mL/L |
| T. Element S2 | 1 | mL/L |

| | | |
|---|---|---|
| T. Element S1 EDTA: 5g/L, FeSO₄: 5g/L T. Element S2 EDTA: 15g/L ZnSO₄-7H₂O: 0.43g/L, CoCl₂-6H₂O: 0.24g/L, MnCl₂-4H₂O: 0.99g/L, CuSO₄-5H₂O: 0.25g/L, NaMoO₄-2H₂O: 0.22g/L, NiCl₂-6H₂O: 0.19g/L, NaSeO₄-10H₂O: 021g/L, H₃BO₄: 0.014g/L | | |

Water temperature: 36°C
Activated sludge: Sludge of anaerobic ammonium oxidizing bacteria cultured in synthetic wastewater in Table 1 (denitrification activity: 2.4 mg-NH₄/g-vss/h)

**[Table 2]**

| | Type of fine powder | Amount of fine powder added (mass%) | Denitrification activity (kg-N/m³/d) |
|---|---|---|---|
| Example 1 | Powdered activated carbon | 1 | 15.0 |
| Example 2 | Magnetite | 2 | 13.5 |
| Comparative Example 1 | - | - | 10.0 |

As shown in Table 2, when the amount of cells of anaerobic ammonium oxidizing bacteria was 1,000 mg/L, the entrapping immobilization pellets of Examples 1 and 2 according to the present invention exhibited denitrification activity higher than in the entrapping immobilization pellets of Comparative Example 1. In particular, the entrapping immobilization pellets of Examples 1 containing powdered activated carbon exhibited highest denitrification activity. Although the entrapping immobilization pellets of Examples 2 containing magnetite exhibited denitrification activity higher than in the entrapping immobilization pellets of Comparative Example 1, magnetite was less effective than powdered activated carbon. It was also confirmed that iron powder can achieve the same effect.

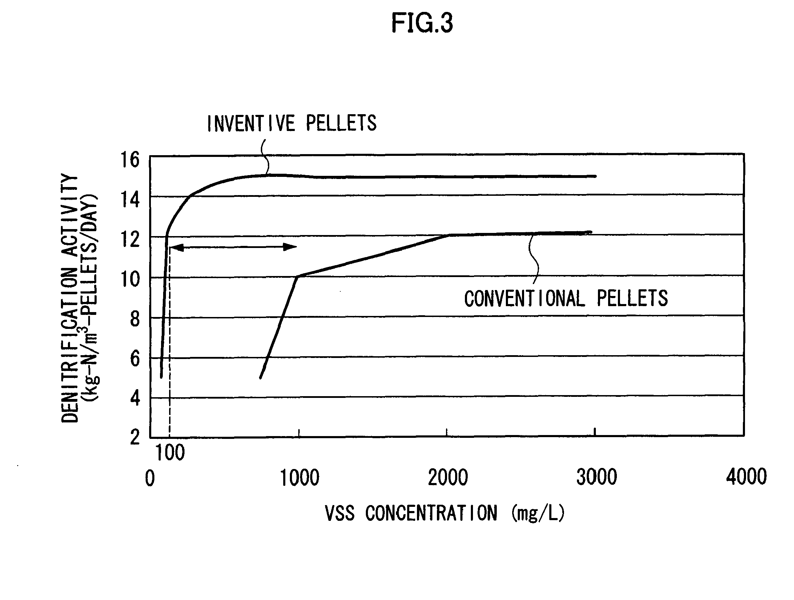

Next, denitrification activity of the entrapping immobilization pellets of Example 1 was determined, where the amount of cells of anaerobic ammonium oxidizing bacteria contained in the entrapping immobilization pellets was changed in the VSS concentration range of 100 mg/L to 3,000 mg/L (Inventive Pellets in Fig. 3). Denitrification activity of the entrapping immobilization pellets of Comparative Example 1 was also determined, where the amount of cells of anaerobic ammonium oxidizing bacteria was changed in the same manner (Conventional Pellets in Fig. 3). The evaluation results at that time are shown in Table 3. In Fig. 3, the horizontal axis indicates the initial VSS concentration (mg/L) corresponding to the amount of cells of anaerobic ammonium oxidizing bacteria contained in the entrapping immobilization pellets.

As is clear from Fig. 3, the entrapping immobilization pellets of Example 1 in which the VSS concentration of anaerobic ammonium oxidizing bacteria was changed in the range of 100 mg/L to 3,000 mg/L exhibited denitrification activity stably higher than in the conventional entrapping immobilization pellets of Comparative Example 1 in which the amount of cells of anaerobic ammonium oxidizing bacteria was changed in the same manner.

In particular, it was found that the entrapping immobilization pellets of Example I had sufficiently high activity even when the amount of cells of anaerobic ammonium oxidizing bacteria corresponded to a low VSS concentration of 1,000 mg/L or less, and that the pellets had activity equal to or higher than in the conventional entrapping immobilization pellets of Comparative Example 1 even when the amount of cells of anaerobic ammonium oxidizing bacteria corresponded to a VSS concentration of 100 mg/L.

On the other hand, the conventional entrapping immobilization pellets of Comparative Example 1 had drastically decreased denitrification activity and almost failed to treat wastewater, when the amount of cells of anaerobic ammonium oxidizing bacteria corresponded to a low VSS concentration of 1,000 mg/L or less.

As described above, it was found that the entrapping immobilization pellets of the present invention exhibit activity equal to or higher than in conventional entrapping immobilization pellets even when the amount of cells of anaerobic ammonium oxidizing bacteria is 10% of that of the conventional entrapping immobilization pellets, and that the present invention can provide entrapping immobilization pellets having activity about 10 times higher than in conventional entrapping immobilization pellets.

The entrapping immobilization pellets of Example 1 were visually observed to have red sludge of anaerobic ammonium oxidizing bacteria uniformly dispersed therein. On the contrary, red sludge of anaerobic ammonium oxidizing bacteria were sedimented and not uniformly dispersed in the entrapping immobilization pellets of Comparative Example 1. Thus, it was confirmed that sedimentation of sludge of anaerobic ammonium oxidizing bacteria when entrapped and immobilized can be inhibited by addition of powdered activated carbon.

Next, the entrapping immobilization pellets of Example 1 were examined for the relation between the amount of powdered activated carbon added and denitrification activity and the relation between the amount of powdered activated carbon added and pellet strength.

The entrapping immobilization pellets were prepared in the same manner as in the above-described Example 1, except that the amount of cells of anaerobic ammonium oxidizing bacteria corresponded to a VSS concentration of 1,000 mg/L and the amount of powdered activated carbon was changed as shown in Table 2.

Pellet strength of the entrapping immobilization pellets (kg/cm²) was calculated by measuring using a rheometer the pressure (kg) gradually applied to the pellet surface enough to break the pellets, and dividing the pressure by the cross-sectional area of the pellets (cm²). Pellet strength was evaluated as "Good" when sufficiently practical, "Fair" when slightly low but practical, and "Poor" when extremely low and not practical. The evaluation results are shown in Table 3.

**[Table 3]**

| | Amount of powdered activated carbon added (mass%) | Pellet strength | Denitrification activity (kg-N/m³/d) |
|---|---|---|---|
| Example 3 | 0.01 | Good | 10.7 |
| Example 4 | 0.05 | Good | 12.0 |
| Example 5 | 0.5 | Good | 15.0 |
| Example 6 | 5 | Good | 15.0 |
| Example 7 | 10 | Fair | 15.0 |

As shown in Table 3, denitrification activity of the entrapping immobilization pellets was obviously improved as compared with the entrapping immobilization pellets of Comparative Example 1 (Conventional Pellets) when the amount of powdered activated carbon added was 0.05 mass% or more, but the denitrification activity was almost the same as that of the entrapping immobilization pellets of Comparative Example 1 (without powdered activated carbon) when the amount of powdered activated carbon added was 0.01 mass%. In particular, when the amount of powdered activated carbon added was 0.5 mass% or more, denitrification activity was not increased any more and showed an almost constant value.

It was also found that pellet strength of the entrapping immobilization pellets is sufficiently practical when the amount of powdered activated carbon added is 5 mass% or less, but the pellet strength is practical but slightly decreased when the amount of powdered activated carbon added is more than 5 mass%. This is presumably because gelling reaction (polymerization reaction) of the immobilizing material tends to be inhibited near the powdered activated carbon.

Thus, it was found that the amount of powdered activated carbon added to the entrapping immobilization pellets is preferably 0.05 mass% to 5 mass%, and more preferably 0.5 mass% to 5 mass% from the viewpoint of denitrification activity and pellet strength.

## Claims

1. Entrapping immobilization pellets (14) in which anaerobic ammonium oxidizing bacteria are entrapped and immobilized in an immobilizing material, **characterized by** comprising:
fine activated carbon powder and/or fine metal powder such as magnetite or iron powder.

2. The entrapping immobilization pellets (14) according to claim 1, wherein there are cells of the anaerobic ammonium oxidizing bacteria at a VSS concentration of 100 mg/L to 3,000 mg/L.

3. The entrapping immobilization pellets (14) according to claim 1 or 2, wherein there are the fine activated carbon powder and/or the fine metal powder such as magnetite or iron powder in an amount of 0.05 mass% to 5 mass% based on the entrapping immobilization pellets.

4. A process for producing entrapping immobilization pellets (14) in which anaerobic ammonium oxidizing bacteria are entrapped and immobilized in an immobilizing material, comprising the steps of:
mixing the anaerobic ammonium oxidizing bacteria with fine activated carbon powder and/or fine metal powder such as magnetite or iron powder, and
mixing the immobilizing material with the mixture to form a gel.

5. The process for producing entrapping immobilization pellets (14) according to claim 4, wherein the entrapping immobilization pellets comprise cells of the anaerobic ammonium oxidizing bacteria at a VSS concentration of 100 mg/L to 3,000 mg/L, and comprise the fine activated carbon powder and/or the fine metal powder such as magnetite or iron powder in an amount of 0.05 mass% to 5 mass% based on the entrapping immobilization pellets.

6. Wastewater treatment equipment (10), comprising:
a biological treatment tank (12) packed with the entrapping immobilization pellets (14) according to any one of claims 1 to 3.
